# EUROPEAN PATENT APPLICATION

(11) **EP 1 535 615 A1**
(43) Date of publication of application: **01.06.2005**
(21) Application number: 03741350.7
(22) Date of filing: 11.07.2003
(51) Int. Cl.: A61K 31/485, A61K 9/14, A61K 9/72, A61K 47/04, A61K 47/12, A61P 25/04

(54) **COMPOSITION FOR NASAL ABSORPTION**

(30) Priority: 11.07.2002 JP 2002203093
(71) Applicant: TAIHO PHARMACEUTICAL CO., LTD., Tokyo 101-0054 (JP)
(72) Inventor: YANAGAWA, Akira, Yokohama-shi, Kanagawa 224-0024 (JP)
(74) Representative: Hartz, Nikolai F., Dr.
(86) International application number: PCT/JP2003/008838
(87) International publication number: WO 2004/006929

(57) **Abstract**

This invention attempts to provide a composition for intranasal administration which has markedly lower risk of developing side effects compared to oral formulation, which promptly exhibits analgesic effects, and which has excellent bioavailability.

The composition for nasal absorption comprises a carrier of calcium carbonate and/or calcium phosphate having an average particle size of 500 µm or less and an effective dose of an opioid analgesic uniformly distributed and attached to the carrier.

## Description

### FIELD OF THE INVENTION

This invention relates to a composition for nasal absorption, and more specifically, to a composition for nasal absorption containing an opioid analgesic wherein the opioid analgesic is highly absorbable into the body, and which has low side effects.

### BACKGROUND OF THE INVENTION

Analgesic effect of an opioid analgesic which specifically binds to an opioid receptor to express strong analgesic action to central nervous system is very strong, and post-surgery pain and cancer pain are typical pains that can be relieved by the use of such opioid analgesic. Opioid analgesics are categorized into narcotics which are controlled under the Narcotic and Psychotropic Control Law and non-narcotics other than such narcotics. This categorization is based on the degree of psychological and physical dependency developed after prolonged administration, and not on the pharmacological action of the opioid analgesic or difference in clinical effects (see Rinsho-to-Kenkyu (Clinical Practice and Research), vol. 78, No. 3 (March, 2001), 481-485).

Opioid analgesics act as an agonist for opioid receptors (including known subtypes µ, κ, δ, σ, ε, and the like) which are abundant in central nervous system, and exhibit strong analgesic effects. Various opioid analgesics are known, and their pharmacological properties are consequence of difference in their affinity for the opioid receptor as well as their activity after binding with the opioid receptor. For example, morphine classified in the category of narcotics is a full agonist for µ receptor, while buprenorphine classified in the category of non-narcotics is a partial agonist for µ receptor with affinity for κ receptor, and with weaker development of tolerance compared to morphine. The analgesic effect of opioid analgesics are extremely strong, and there is no reason to refrain from using such opioid analgesics as long as their side effects are well managed with full understanding and effective measures.

Incidentally, a chief complaint of about 70% of the patients suffering from terminal cancer is cancer pain, and an opioid analgesic, especially morphine hydrochloride known as a narcotic analgesic, has been used for a long time in order to relieve the patient from such cancer pain. Pain control of cancer patients is recently carried out in a systematic way, and use of an slow-release oral formulation as a medical morphine formulation has become prevalent.

Typical slow-release oral formulations include a morphine sulfate slow-release drug (trade name: MS-Contin (registered trademark)) which is orally administered twice a day, and a morphine sulfate slow-release drug (trade name: Kadian (registered trademark)) which is orally administered once a day. The pain relief is found to be achievable approximately 80% on average by the administration of these formulations, and use of such formulations have surely contributed for the relief of terminal cancer patients from the torture of cancer pain.

These slow-release oral formulations, however, are not free from problems. The incident rate of side effects caused by use of MS-Contin and Kadian amounts to about 50%. The side effects caused by these drugs are mostly involved in digestive system, for example, constipation, nausea, emesis, thirst, and anorexia. They also cause side effects on central nervous system such as drowsiness and confusion (Yakkyoku (Pharmacy) Vol. 53, No.4 (2002)).

With regard to the administration of analgesic for cancer pain treatment, it is difficult to satisfy the patient only by the "basic analgesic" which is designed to suppress the gentle ebb and flow of continuous pain lasting all the day, and prescription of an "extra fast-acting analgesic" for the unpredictable incident pain separately from the "basic analgesic" is important. The patient then needs to immediately take the medicine on the bases of the patient's own judgement. Prescription of such "extra fast-acting analgesic" is extremely important because patients are prone to fear the unpredictable incident pain not only at the time they are attacked by the incident pain (also referred to as breakthrough pain since the pain becomes even stronger after such pain) one after another, but also at the time they are relieved from the pain almost all the day by the sole use of the "basic analgesic". As described above, opioid therapy of the cancer pain, especially the cancer pain whose main pain generation mechanism is nociceptive pain is considered to be feasible by the combination of periodical intake of the opioid preparation which exhibits stable analgesic effect for a prolonged time and occasional intake of the fast-acting opioid preparation, and the opioid therapy can not be accomplished in the absence of either type of preparation. (See Rinsho-to-Yakubutsu-Chiryo (Clinical Practice and Medication) (2002), Vol.21, No.10, 1032-1038) ).

According to "Cancer Pain Relief" advocated by World Health Organization (WHO), objects of the cancer pain treatment are intended primarily to ensure good sleep without being intervened by the pain, secondly to eliminate pain during the rest in daytime, and thirdly to eliminate pain during motion. In consideration of such objects, administration of the analgesic is generally accomplished on the bases of oral administration to maintain an effective blood level for development of an analgesic action, and the analgesic is administered by transrectal, subcutaneous, or intravenous administration when such oral administration can not be conducted. More specifically, while the analgesic is periodically administered to maintain the effective blood concentration level, side effects of morphine in the early phase of the administration are serious nausea and emesis, and continuous administration of morphine becomes difficult when the patient suffers from such nausea and emesis in the initial administration.

Parenteral administration is also conducted in the case of cancer of digestive system since oral administration is often difficult in such cancer. Many patients, however, feel reluctant about transrectal administration (suppository), while subcutaneous and intravenous administrations are associated with the pain of injection and the problem of muscular atrophy of the administration site after repeated injection. Accordingly, there is a demand for development of opioid analgesics free from such problems. Side effects such as nausea and emesis have been disregarded in the consideration of the usefulness of the main action, namely, the analgesic action, and no constructive efforts have been made for the development of the opioid analgesic preparation with reduced side effects.

The inventors of the present invention have been engaged in the investigation of new administration routes for the medicament which could not be administered in oral route, and one such route has been the transnasal administration. More specifically, nasal cavity which is the site used for administration in the transnasal route has nasal proper lamina including a well-developed venous plexus, and absorption of the medicament through such nasal mucosa and entrance of the medicament into the systemic circulation system have been confirmed.

The inventors of the present invention have also confirmed that such transnasal administration is an excellent approach in suppressing the side effect development of the medicament. The inventors have proposed various carriers which are adapted for such transnasal administration and exhibit sufficient absorption into the body as well as reduced nasal stimulation.

For example, Japanese Patent Application Laid-Open No. 11-322582 discloses a carrier for a medicament which is to be absorbed through the nose, and this carrier has numerous voids, a surface area of 0. 1 to 0. 4 m²/g, a specific weight of about 0. 5 to 1. 0, and a grain size of 15 to 300 µm. Therein it is also disclosed that good absorption of the medicament loaded on the carrier by the body is achievable by this carrier since the medicament can never reach the lung, and the carrier remains attached to the nasal mucosa without becoming detached therefrom by the action of the gravity. Japanese Patent Application Laid-Open No. 8-27031 discloses a composition for nasal absorption which comprises a polyvalent metal compound carrier having an average particle size up to 250 µm and a physiologically active medicament having a molecular weight of up to 40000 attached to the carrier. These patent applications are utterly silent about the opioid analgesic which is the medicament used in the present invention.

The patent applications as mentioned above disclose quite a variety of polyvalent metal compound carriers. These patent applications, however, are utterly silent about the preferability of the calcium carbonate and/or the calcium phosphate for use as a carrier of an opioid transnasal preparation due to their capability of holding the opioid, releasing the opioid by dissolution, and retention in the administration site, and the like. The inventors have also found that some of the carriers know as preferable for use, such as calcium lactate, magnesium stearate, aluminum hydroxide, magnesium oxide, as carriers in the opioid transnasal preparation due to their dissolution upon contact with the moisture in the nasal cavity after the intranasal administration, excessive stimulation of the nasal mucosa, and the like.

### DETAILED DESCRIPTION OF THE INVENTION

In order to enable administration of an opioid analgesic with reduced side effects, the inventors of the present invention conducted an investigation by focusing on the approach of transnasal administration. More specifically, the inventors postulated that the side effects of opioid analgesics as represented by morphine hydrochloride with frequent development of side effects may be reduced by administering the analgesic in transnasal route, and in such intent, examined extensive transnasal formulations that had been prepared.

As a result of such examination, the inventors have unexpectedly found that absorption into the body higher than that of the oral administration can be attained by administering an opioid analgesic after loading on some particular polyvalent metal compound carriers, and that transnasal administration route exhibits steeper rise of the absorption curve after the administration compared to the case of oral administration as well as excellent bioavailability.

The inventors have also found that some opioid analgesics exhibit absorption into the body after the transnasal administration comparable to that of the administration by intravenous route. Such absorption into the body higher than that of the oral administration means that the medicament can be administered at a lower dose, and therefore, the possibility of reducing the frequency of side effect development. More specifically, the prompt rise in the absorption into the body and the good bioavailability attained by the transnasal administration enables use of this administration route in treating not only the continuous pain of the cancer but also the incident pain, and this invention is of great benefit to patients suffering from serious cancer pain. In view of the situation as described above, the present invention provides a composition for nasal absorption comprising a carrier of calcium carbonate and/or calcium phosphate having an average particle size of up to 500 µm and an effective dose of an opioid analgesic uniformly distributed and attached to the carrier.

The present invention also provides use of a composition for nasal absorption in producing an analgesic for post-surgery pain or cancer pain, wherein the composition comprises a carrier of calcium carbonate and/or calcium phosphate having an average particle size of up to 500 µm and an effective dose of an opioid analgesic uniformly distributed and attached to the carrier.

The present invention also provides a method for treating post-surgery pain or cancer pain. This method comprises the step of intranasally administering a composition comprising a carrier of calcium carbonate and/or calcium phosphate having an average particle size of up to 500 µm and an effective dose of an opioid analgesic uniformly distributed and attached to the carrier.

In other words, a characteristic feature of the present invention is the excellent absorption into the body attained by selecting the calcium carbonate and/or the calcium phosphate from various polyvalent metal compounds proposed by the inventors of the present invention that has been used for the carrier adapted for nasal absorption, uniformly distributing and attaching the narcotic or non-narcotic opioid analgesic to the thus selected carrier, and intranasally administering the analgesic.

### BRIEF DESCRIPTION OF DRAWINGS

FIG. 1 is a graph showing kinetics of plasma morphine concentration measured in Test Examples 1 to 3 of the present invention and the Comparative Example in relation to time.
FIGS. 2 and 3 are graphs showing kinetics of plasma buprenorphine concentration measured in Test Example 4 of the present invention in relation to time.
FIG. 4 is a graph showing plasma fentanyl concentration measured in Test Example 5 of the present invention in relation to time.

### BEST MODE FOR CARRYING OUT THE INVENTION

The opioid analgesic used in the composition for nasal absorption of the present invention include both narcotic and non-narcotic opioid analgesics. The narcotic opioid analgesics are opioid analgesics controlled under the Narcotic and Psychotropic Control Law, and the narcotic opioid analgesic may be either a natural narcotic or a synthetic narcotic. The non-narcotic opioid analgesics are analgesics with analgesic effects, which are less likely to invite physical and pchycological dependence or habituation after prolonged administration compared to the narcotic analgesics. These analgesics are used mainly in the form of an oral or injection preparation in order to ameliorate cancer pain of the terminal cancer patients.

Examples of such opioid analgesic used in the present invention include a narcotic opioid analgesic selected from opium, opium/ipecac preparation, morphine, morphine/atropine preparation, opium alkaloids preparation, opium alkaloids/atropine preparation, opium alkaloids/scopolamin preparation, ethylmorphine, oxycodon, oxycodon/atropine preparation, pethidine, pethidine/levallorphan preparation, codeine, dihydrocodeine, fentanyl, droperidol/fentanyl preparation, oxymetebanol, levorphanol, propoxyphene, methadone, hydromorphone, and meperidine; a non-narcotic opioid analgesic selected from buprenorphine, butorphanol, pentazocine, pentazocine/naloxone preparation, dezocine, tramadol, and eptazocine; and a pharmaceutically acceptable salt thereof.

The pharmaceutically acceptable salt used in the present invention is preferably an acid addition salt with a pharmaceutically acceptable acid such as a salt with an inorganic acid such as hydrochloric acid, sulfuric acid, phosphoric acid, and hydrobromic acid or an organic acid such as oxalic acid, maleic acid, fumaric acid, malic acid, tartaric acid, citric acid, benzoic acid, acetic acid, lactic acid, p-toluenesulfonic acid, or methanesulfonic acid. More preferably, the pharmaceutically acceptable salt is a salt with hydrochloric acid, sulfuric acid, phosphoric acid, hydrobromic acid, tartaric acid, or citric acid.

Typical opioid analgesics include a narcotic opioid analgesic selected from opium, opium/ipecac preparation, morphine hydrochloride, morphine sulfate, morphine hydrochloride/atropine sulfate preparation, opium alkaloids hydrochloride preparation, opium alkaloids hydrochloride/atropine sulfate preparation, opium alkaloids hydrochlorides/scopolamine hydrobromide preparation, ethylmorphine hydrochloride, compound oxycodone (oxycodone hydrochloride/hydrocotarnine hydrochloride), compound oxycodone/atropine sulfate preparation, pethidine hydrochloride, pethidine hydrochloride/levallorphan tartrate preparation, codeine phosphate, dihydrocodeine phosphate, fentanyl, fentanyl citrate, droperidol/fentanyl citrate preparation, oxymetebanol, levorphanol, propoxyphene, methadone, hydromorphone and meperidine; and a non-narcotic opioid analgesic selected from buprenorphine hydrochloride, butorphanol tartrate, pentazocine, pentazocine hydrochloride, pentazocine hydrochloride/naloxone preparation, dezocine, tramadol hydrochloride, and eptazocine hydrobromide.

More preferable opioid analgesics include morphine, fentanyl, buprenorphine, and their acid addition salts. Typical examples of such preferable opioid analgesics include morphine hydrochloride, morphine sulfate, morphine hydrochloride/atropine sulfate preparation, fentanyl, fentanyl citrate, droperidol/fentanyl citrate preparation, and buprenorphine hydrochloride.

The opioid analgesics as described above are used in oral, injection, or suppository formulation, and use of such opioid analgesics by transnasal administration using a fine powder carrier has never been investigated. In addition, the opioid analgesics are associated with the undesirable problems of habituation, addiction, and the like after their prolonged use, and strict limitation was set on their use. Accordingly, investigation of their formulation into a drug product has been difficult.

The carrier used in the composition for nasal absorption provided by the present invention is calcium carbonate and/or calcium phosphate. Use of a water soluble has been deemed adequate in conventional intranasal formulations in view of the absorption of the effective ingredient in the body. However, according to the findings by the inventors of the present invention, a water soluble carrier is not necessary favorable, and a carrier like calcium carbonate and/or calcium phosphate used in the present invention which becomes attached to the nasal mucosa without dissolving in water is capable of efficiently releasing the opioid analgesic to attain the biological absorption of the effective ingredient from the nasal mucosa.

Typical conventional carriers used include calcium carbonate, calcium phosphate, calcium lactate, calcium stearate, aluminum hydroxide, aluminum oxide, magnesium carbonate, and magnesium hydroxide. Of these carriers, calcium lactate becomes dissolved on the nasal mucosa, and calcium stearate turns into an oily form on the nasal mucosa, and these compounds are less adequate for use as a carrier. In addition, aluminum hydroxide, aluminum oxide, magnesium carbonate, and magnesium hydroxide are highly stimulative, and use of these compounds for the carrier in an intranasal formulation may present various problems.

In contrast, calcium carbonate and/or calcium phosphate used in the present invention has been found to be particularly favorable since these compounds exhibit good affinity for opioid analgesics, smooth attaching and releasing of the opioid analgesic, and little stimulation for the nasal mucosa. Among these, calcium carbonate is particularly preferable.

The calcium carbonate and/or calcium phosphate used for the carrier in the present invention may be used either alone or in combination.

Use of the carrier having an average particle size of up to 500 µm, preferably up to 250 µm, more preferably up to 100 µm, and most preferably up to 20 to 100 µm was found to be favorable.

Accordingly, an embodiment of the present invention is a composition for nasal absorption comprising a carrier of calcium carbonate and/or calcium phosphate having an average particle size of 20 to 100 µm and an opioid analgesic uniformly distributed and attached to the carrier.

Among such embodiment of the present invention, a typical embodiment is the composition for nasal absorption wherein the opioid analgesic is morphine hydrochloride, morphine sulfate, morphine hydrochloride/atropine sulfate preparation, fentanyl, fentanyl citrate, droperidol/fentanyl citrate preparation, or buprenorphine hydrochloride, and the carrier is calcium carbonate.

Examples of such embodiments are (1) a composition for nasal absorption comprising a carrier of calcium carbonate having an average particle size of 20 to 100 µm and an opioid analgesic uniformly distributed and attached to the carrier wherein the opioid analgesic is morphine or its acid addition salt; (2) a composition for nasal absorption comprising a carrier of calcium carbonate having an average particle size of 20 to 100 µm and an opioid analgesic uniformly distributed and attached to the carrier wherein the opioid analgesic is buprenorphine or its acid addition salt; and (3) a composition for nasal absorption comprising a carrier of calcium carbonate having an average particle size of 20 to 100 µm and an opioid analgesic uniformly distributed and attached to the carrier wherein the opioid analgesic is fentanyl or its acid addition salt. The present invention is effective for treating pains such as pain after surgery or cancer pain, particularly in treating incident pain and/or continuous pain of the cancer pain.

The amount of the opioid analgesic included in the composition provided by the present invention is the effective dose of the opioid analgesic, and this effective dose varies depending on the type of the analgesic selected, the disease treated, desired frequency of the administration, effects to be attained by the administration, and the like. When the composition of the present invention is used by intranasal administration, the effects realized by the formulation containing the active substance may be determined by comparing the bioavailability with other known formulations.

Typical oral administration dose of an opioid analgesic is, for example, 10, 30, and 60 mg/tablet in the case of morphine hydrochloride. However, when the composition for nasal absorption provided by the present invention is intranasally administered, the active component is more promptly absorbed by the body at a higher bioavailability compared to such oral administration as demonstrated in the following Examples. Accordingly, the dose administered in the present invention in single administration may be lower than the dose determined by such content of the active component in the oral administration.

Amount of the opioid analgesic incorporated in the composition of the present invention may vary depending on the weight of the formulation. Typical content, however, is about 0.01 to 50%, and preferably about 0.02 to 40% in 100% by weight of the formulation.

Amount of the calcium carbonate and/or calcium phosphate used as a carrier in the composition for nasal absorption of the present invention may also vary depending on the weight of the formulation. Typical content, however, is about 50 to 99. 99%, and preferably about 40 to 99. 98% in 100% by weight of the formulation.

The composition for nasal absorption provided by the present invention may also include known additives such as lubricant, binder, diluent, colorant, preservative, antiseptic, and corrective as desired in order to improve physical properties, appearance, or odor of the formulation. Examples of the lubricants include talc, stearic acid and a salt thereof, and wax, and exemplary binders include starch, dextrin, gelatin, and hydroxypropylcellulose. Examples of the diluents include starch and lactose, and exemplary colorants include Red No. 2. Examples of the preservatives include ascorbic acid, examples of the antiseptics include paraoxybenzoate, and examples of the correctives include menthol.

Of the examples as mentioned above, the binder incorporated in the composition is preferably a water soluble polymer such as starch, dextrin, gelatin, hydroxypropylcellulose, and a typical example of such water soluble polymer is rice powder. The starch, dextrin, gelatin, hydroxypropylcellulose, or other water soluble polymer compound as typified by the rice powder contributes for the good binding between the carrier and the opioid analgesic in the composition for nasal absorption provided by the present invention, and such water soluble polymer compound also functions as a bumper during the freeze drying in the production of such composition.

The composition for nasal absorption provided by the present invention containing an effective dose of the opioid analgesic can be produced by mixing the carrier specifically adapted for use in the present invention, namely, physiologically acceptable calcium carbonate and/or calcium phosphate in powder or crystalline form having an average particle size of up to 500 µm with the effective ingredient, namely, the opioid analgesic.

The mixing is typically accomplished by applying pressure or shear stress, for example, by mixing the components in a mortar.

The carrier used in the production of the composition for nasal absorption of the present invention may have an average particle size of up to 250 µm, preferably up to 100 µm, and more preferably 20 to 100 µm. In the meanwhile, the opioid analgesic is also preferably in the form of a fine powder with an average particle size of typically up to 20 µm, and preferably up to 10 µm.

More specifically, an effective dose of the opioid analgesic is mixed with a solution containing about 0.1 1 to 10% by weight, and preferably about 1 to 5% by weight of a binder such as starch as desired, and this mixture solution is freeze dried. The freeze dried powder is then kneaded with the carrier of the present invention at a kneading moisture of about 55% to obtain the composition for nasal absorption in the form of a fine powder comprising the carrier and the opioid analgesic uniformly distributed and attached to the carrier.

Alternatively, the composition for nasal absorption of the present invention may be produced by dispersing an effective dose of the opioid analgesic and the carrier optionally with the binder in water, and freeze drying the dispersion.

The production method, however, is not limited to those described above, and various changes and modifications may be made to such method.

The resulting composition for nasal absorption may be filled in a low-grease type capsule to prevent loss of the active substance before its use (for example, before the intranasal administration), and the capsule may be placed in an adequate package, preferably, sealed package. An exemple of such sealed package is a combination of blister pack and aluminum backing. In this case, the moisture is preferably kept at 60% or less throughout the steps.

### Examples

Next, the effects specifically attained by the composition for nasal absorption of the present invention are demonstrated by the following Test Examples.

### Test Example 1

The composition for nasal absorption of the present invention was prepared by using morphine hydrochloride for the opioid analgesic and calcium carbonate for the carrier. More specifically, 2. 0 mg of morphine hydrochloride (using morphine hydrochloride powder "Sankyo"), 37. 2 mg of calcium carbonate, and 0.4 mg of rice powder were mixed, stirred, and allowed to stand for 5 minutes. After adding an adequate amount of purified water for freeze drying, the mixture was kneaded and freeze dried under the condition of about -40°C by using a freeze dryer (manufactured by EVAC). After the freeze drying, the product was gradually warmed to 25°C in about 4 hours, and 0.4 mg of calcium stearate was added as a lubricant. Total weight of the formulation was 40. 0 mg.

The calcium carbonate used for the carrier had an average particle size of about 62 µm.

A group of 3 male rhesus monkeys (body weight, 3.35 to 4. 05 kg) were transnasally administered with 40. 0 mg of the composition as described above (single dose), and the blood (about 2 mL) was collected from cephalic vein or saphenous vein at 0, 5, 10, 20, 30, 60, 120, and 240 minutes after the administration to measure the amount of morphine (in terms of morphine hydrochloride) in plasma by liquid chromatography coupled with mass spectrometry (LC-MS/MS).

Transnasal administration was conducted by using a device designed for administering a transnasal absorptive powder formulation manufactured by UNISIA JECS CO. (JetRiser for monkeys).

In Comparative Example 1, 2.0 mg of morphine hydrochloride (using morphine hydrochloride powder "Sankyo") was filled in a capsule, and the capsule was orally administered. The blood was collected as in the case of Test Example 1 to measure the.amount of morphine (in terms of morphine hydrochloride) in plasma by LC-MS/MS.

The results are summarized in Table 1.

**Table 1:**

| Plasma morphine concentration (in terms of morphine hydrochloride), unit: ng/mL | | | | | | |
|---|---|---|---|---|---|---|
| Time (min) | Test Example 1 (Transnasal administration) Morphine HCl 2mg/40mg (with rice powder) | | | Comparative Example 1 (Oral administration) Morphine HCl 2mg | | |
| | Animal 1 | Animal 2 | Animal 3 | Animal 1 | Animal 2 | Animal 3 |
| 0 | _(Note) | - | - | - | - | - |
| 5 | 15.9 | - | 15.8 | - | - | - |
| 10 | 13.0 | 8.2 | 21.3 | - | - | - |
| 20 | 14.2 | 6.0 | 19.4 | - | - | - |
| 30 | 15.7 | 5.8 | 18.9 | - | - | - |
| 60 | 13.5 | - | 23.1 | 5.0 | - | 11.0 |
| 120 | 9.1 | 5.4 | 17.1 | - | - | - |
| 240 | 4.6 | - | 8.0 | - | - | - |
| Note: "-" indicates that the concentration was under the detection limit (4.3 ng). | | | | | | |

### Test Examples 2 and 3

The composition for nasal absorption of the present invention was prepared by using morphine hydrochloride for the opioid analgesic and calcium carbonate for the carrier. The particular method used for the production was similar to the one used in Test Example 1.

The composition used in Test Example 2 was 2.0 mg of morphine hydrochloride, 37.6 mg of calcium carbonate, and 0. 4 mg of calcium stearate (total weight of the formulation, 40. 0 mg), and the composition used in Test Example 3 was 8. 0 mg of morphine hydrochloride, 148.8 mg of calcium carbonate, 1. 6 mg of rice powder, and 1. 6 mg of calcium stearate, which are four times that of Test Example 1, (total weight of the formulation, 160.0 mg).

The calcium carbonate used for the carrier had an average particle size of about 62 µm.

The male rhesus monkeys (body weight 3. 35 to 4. 05 kg) used in Test Example 1 were also used in Test Examples 2 and 3. A group of three monkeys in Test Example 2, and a group of two monkeys in Test Example 3 were used, and the compositions as described above were transnasally administered in single dose. The blood (about 2 mL) was collected from cephalic vein or saphenous vein at 0, 5, 10, 20, 30, 60, 120, and 240 minutes after the administration to measure the amount of morphine (in terms of morphine hydrochloride) in plasma by LC-MS/MS.

Transnasal administration was conducted by using a device designed for administering a transnasal absorptive powder formulation manufactured by UNISIA JECS CO. (JetRiser for monkeys).

The results are summarized in Table 2.

**Table 2:**

| Plasma morphine concentration (morphine hydrochloride) unit: ng/mL | | | | | |
|---|---|---|---|---|---|
| Time (min) | Test Example 2 (Transnasal administration) Morphine HCl 2mg/40mg (without rice powder) | | | Test Example 3 (Transnasal administration) Morphine HCl 8mg/160mg (with rice powder) | |
| | Animal 1 | Animal 2 | Animal 3 | Animal 2 | Animal 3 |
| 0 | - | - | - | - | - |
| 5 | 11.1 | 17.5 | 4.6 | 75.8 | 69.5 |
| 10 | 20.3 | 12.2 | 8.7 | 85.0 | 96.2 |
| 20 | 17.9 | 12.3 | 14.7 | 65.2 | 89.2 |
| 30 | 16.9 | 13.0 | 19.3 | 62.4 | 88.0 |
| 60 | 12.4 | 10.4 | 21.7 | 46.3 | 67.7 |
| 120 | 9.2 | 6.2 | 13.4 | 23.6 | 38.4 |
| 240 | - | - | 7.0 | 6.6 | 17.6 |

Elimination rate constant (Kel) was calculated from depuration phase data of the plasma concentration measured in Test Examples 1 to 3 by a method (residual method) not depending on the model using linear least squares approach, and half life (T_{1/2}) was calculated by the equation: T_{1/2} = Ln (2) /Kel. Maximum blood concentration (Cₘₐₓ) was determined from the measured data, and area under the plasma concentration-time curve (AUC) in the administration period (t) was calculated in terms of the value obtained by trapezoidal rule. The average values of these pharmacokinetic parameters are compared in Table 3, below.

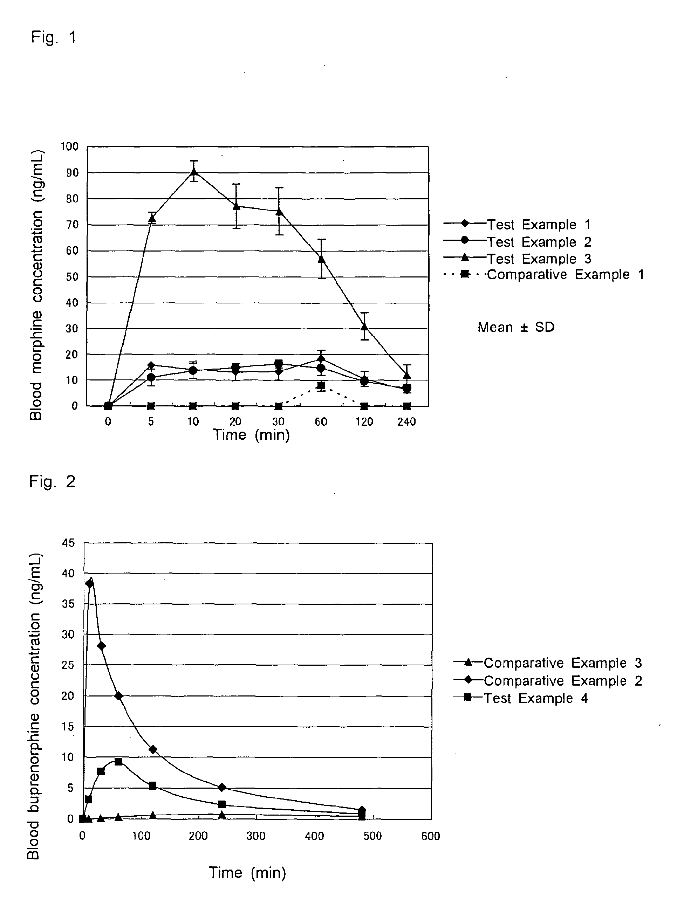

FIG. 1 is a graph showing kinetics of the plasma morphine concentration measured in Test Examples 1 to 3 and Comparative Example in relation to the time.

**Table 3:**

| Pharmacokinetic parameters | | | | |
|---|---|---|---|---|
| Time (min) | Comparative Example 1 Oral administration 2 mg | Transnasal administration | | |
| | | Test Example 1 Morphine HCl 2 mg/40 mg (with rice powder) | Test Example 2 Morphine HCl 2 mg/40 mg (without rice powder) | Test Example 3 Morphine HCl 8mg/160mg (with rice powder) |
| Cₘₐₓ (ng/mL) | 5.3±5.5 | 15.7±7.5 | 19.8±2.1 | 90.6±7.92 |
| T_{1/2} (hr) | - | 1.94 | 1.66±0.27 | 1.32±0.34 |
| AUC(0-4) (hr^{.}ng/mL) | - | 38.2±26.2 | 33.3±17.9 | 156.7±44.34 |

As evident from the results shown in Tables 1 to 3 and FIG. 1, the compositions for nasal absorption of the present invention were confirmed to exhibit prompt absorption at 5 to 10 minutes after the administration, and a blood concentration of 10 to 20 ng/mL or higher which is the level of blood analgesic concentration deemed to be effective in human is continuously attained.

In Test Examples 1 to 3 of the present invention, the analgesic and sedation effects were also evaluated by pinching skin and muscle of the rhesus monkey with forceps at a regular time interval and observing the reaction of the animal.

The analgesic effect was confirmed in animals 1 and 2 of Test Example 1 at 30 to 60 minutes after the administration, and animal 3 of Test Example 1 at 60 minutes after the administration since these animals no longer reacted to the pinching by the forceps.

These results demonstrate the usefulness of the composition for nasal absorption of the present invention.

Comparison of the pharmacokinetic parameters revealed improved absorption promoting action of the present invention. Compared to the oral administration of 2 mg of morphine hydrochloride, administration of the composition for nasal absorption of the same amount (2 mg) had absorption promoting action of 3 to 4 folds in Cₘₐₓ, and about 15 to 20 folds in AUC.

### Test Example 4

The composition for nasal absorption of the present invention was prepared by using buprenorphine hydrochloride for the opioid analgesic and calcium carbonate and calcium stearate for the carrier. More specifically, 10.8 mg of buprenorphine hydrochloride (manufactured by MACFARLAN SMITH) which had been crushed in a agate mortar and shifted through a 100 mesh screen, and 1969.2 mg of calcium carbonate were mixed, and an adequate amount of purified water was added. After kneading the mixture, the mixture was freeze dried under the condition of about -40°C by using a freeze dryer (manufactured by Kyowa Vacuum Technologies). After the freeze drying, the product was gradually warmed to 25°C in about 4 hours to give 1961. 9 mg of the freeze dried product. To this product was added 19. 6 mg of calcium stearate as a lubricant, and the mixture was shifted through a 83 mesh screen. Total weight of the formulation was 1980.2 mg. The calcium carbonate used for the carrier had an average particle size of about 46 µm.

It is to be noted that 10. 8 mg of buprenorphine hydrochloride corresponds to 10. 0 mg of buprenorphine.

A group of 3 male cynomolgus monkeys (body weight, 2. 60 to 3. 00 kg) were transnasally administered in single dose with the composition as described above at a dose in terms of buprenorphine of 0. 1 mg/kg. The blood (about 2 mL) was collected from cephalic vein or saphenous vein at 0, 10, 30, 60, 120, 240, and 480 minutes after the administration to measure the amount of buprenorphine in plasma by liquid chromatography coupled with mass spectrometry (LC-MS/MS).

Transnasal administration was conducted by using a device designed for administering a transnasal absorptive powder formulation manufactured by UNISIA JECS CO. (JetRiser for monkeys).

As a Comparative Example, buprenorphine hydrochloride at a dose of 0.1 mg/kg in terms of buprenorphine was intravenously administered. The blood was collected as in the case of the Test Example to measure the amount of buprenorphine in plasma by LC-MS/MS.

Also, a suppository of buprenorphine (Lepetan (suppository) manufactured Otsuka Pharmaceutical Co., Ltd.) was intrarectally administered at a dose of 0.1 mg/kg in terms of buprenorphine. The blood was collected as in the case of the Test Example to measure the amount of buprenorphine in plasma by LC-MS/MS.

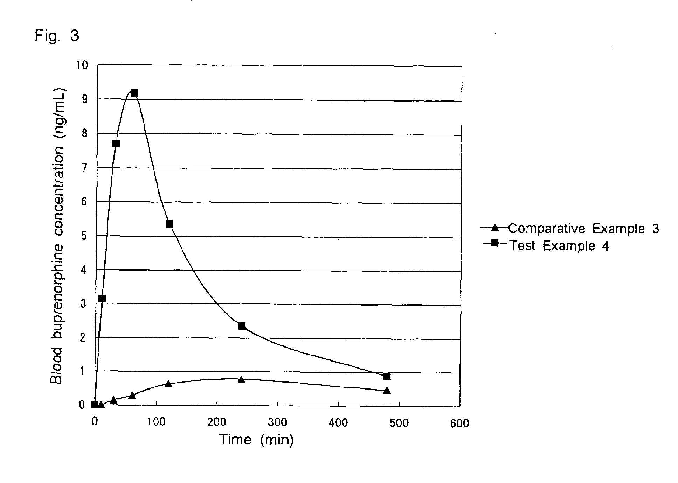

The results are respectively shown in the following Table 4 (Test Example 4: transnasal administration), Table 5 (Comparative Example 2: intravenous administration), and Table 6 (Comparative Example 3: intrarectal administration). The results are also shown in the form of graphs in FIGS. 2 and 3.

**Table 4:**

| Plasma buprenorphine concentration (unit: ng/mL) | | | | |
|---|---|---|---|---|
| Time (min) | Test Example 4 (Transnasal administration) | | | |
| | Animal 1 | Animal 2 | Animal 3 | Mean±SD |
| 0 | - | - | - | 0 |
| 10 | 2.93 | 2.39 | 4.1 | 3.14±0.87 |
| 30 | 6.03 | 7.38 | 9.66 | 7.69±1.83 |
| 60 | 8.74 | 7.02 | 11.8 | 9.19±2.42 |
| 120 | 6.15 | 4.21 | 5.7 | 5.35±1.01 |
| 240 | 3.14 | 1.63 | 2.28 | 2.35±0.76 |
| 480 | 1.42 | 0.46 | 0.75 | 0.88±0.49 |

**Table 5:**

| Plasma buprenorphine concentration (unit: ng/mL) | | | | |
|---|---|---|---|---|
| Time (min) | Comparative Example 2 (Intravenous administration) | | | |
| | Animal 1 | Animal 2 | Animal 3 | mean±SD |
| 0 | - | - | - | 0 |
| 10 | 41.40 | 32.16 | 41.33 | 38.30±5.31 |
| 30 | 32.83 | 18.02 | 33.53 | 28.12±8.76 |
| 60 | 16.85 | 16.45 | 26.51 | 19.94±5.70 |
| 120 | 8.27 | 9.34 | 16.13 | 11.25±4.26 |
| 240 | 3.78 | 4.55 | 7.05 | 5.13±1.71 |
| 480 | 1.46 | 0.97 | 2.00 | 1.48±0.52 |

**Table 6:**

| Plasma buprenorphine concentration (unit: ng/mL) | | | | |
|---|---|---|---|---|
| Time (min) | Comparative Example 3 (Intrarectal administration) | | | |
| | Animal 1 | Animal 2 | Animal 3 | mean±SD |
| 0 | - | - | - | 0 |
| 10 | - | - | - | 0 |
| 30 | 0.13 | 0.19 | 0.11 | 0.14±0.04 |
| 60 | 0.18 | 0.53 | 0.16 | 0.29±0.21 |
| 120 | 0.58 | 1.12 | 0.19 | 0.63±0.47 |
| 240 | 0.15 | 1.40 | - | 0.77±0.89 |
| 480 | - | 0.47 | - | 0.47 |

As evident from the results shown in Tables 4 to 6, the transnasal formulation of the present invention were confirmed to exhibit prompt absorption at 5 to 10 minutes after the administration, and the blood concentration was remarkably higher than the commercially available suppository preparation of buprenorphine hydrochloride (Lepetan, suppository). The blood concentration was about 1/4 of the intravenous administration, indicating the favorable absorption into the body. The nasal absorption formulation of the present invention was also confirmed to exhibit an analgesically effective blood concentration for a prolonged period.

### Test Example 5:

The composition for nasal absorption of the present invention was prepared by using fentanyl citrate for the opioid analgesic and calcium carbonate and calcium stearate for the carrier. More specifically, 2.073 mg of fentanyl citrate and 344. 6 mg of calcium carbonate were mixed, stirred, and allowed to stand for 5 minutes. After adding an adequate amount of purified water for freeze drying, the mixture was kneaded and freeze dried under the condition of about -40°C by using a freeze dryer (manufactured by Kyowa Vacuum Technologies). After the freeze drying, the product was gradually warmed to 25°C in about 4 hours, and 3.503 mg of calcium stearate was added as a lubricant. Total weight of the formulation was 350.2 mg. The calcium carbonate used for the carrier had an average particle size of about 65 µm.

A group of 3 male cynomolgus monkeys (body weight, 2. 60 to 3.30 kg) were transnasally administered in single dose with the composition as described above at a dose in terms of fentanyl of 0. 03 mg/kg. The blood (about 2 mL) was collected from cephalic vein or saphenous vein at 0, 10, 30, 60, 120, 240, and 480 minutes after the administration to measure the amount of fentanyl in plasma by liquid chromatography coupled with mass spectrometry (LC-MS/MS).

Transnasal administration was conducted by using a device designed for administering a transnasal absorptive powder formulation manufactured by UNISIA JECS CO. (JetRiser for monkeys).

As a Comparative Example, fentanyl citrate at a dose of 0.03 mg/kg in terms of fentanyl was intravenously administered. The blood was collected as in the case of the Test Example to measure the amount of fentanyl in plasma by LC-MS/MS.

The results are respectively shown in the following Table 7 (Test Example 5: transnasal administration) and Table 8 (Comparative Example 4: intravenous administration).

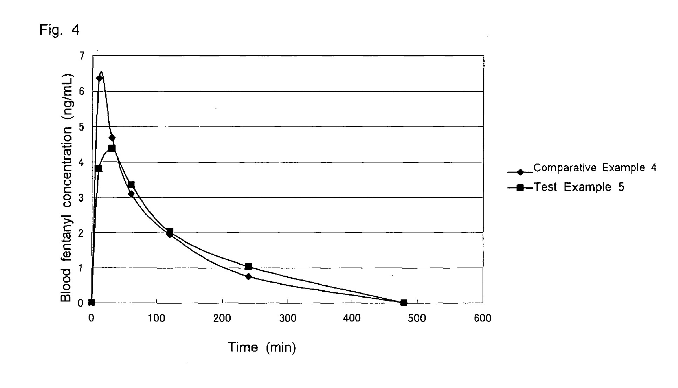

The kinetics are shown in FIG. 4.

**Table 7:**

| Plasma fentanyl concentration (unit: ng/mL) | | | | |
|---|---|---|---|---|
| Time (min) | Test Example 5 (Transnasal administration) | | | |
| | Animal 1 | Animal 2 | Animal 3 | Mean±SD |
| 0 | - | - | - | 0 |
| 10 | 2.748 | 4.461 | 4.215 | 3.808±0.926 |
| 30 | 3.076 | 4.239 | 5.827 | 4.381±1.381 |
| 60 | 2.121 | 3.907 | 4.050 | 3.359±1.075 |
| 120 | 1.361 | 2.598 | 2.120 | 2.026±0.624 |
| 240 | 0.810 | 0.879 | 1.425 | 1.038±0.337 |
| 480 | - | - | - | 0 |

**Table 8:**

| Plasma fentanyl concentration (unit: ng/mL) | | | | |
|---|---|---|---|---|
| Time (min) | Comparative Example 4 (Intravenous administration) | | | |
| | Animal 1 | Animal 2 | Animal 3 | Mean±SD |
| 0 | - | - | - | 0 |
| 10 | 5.321 | 7.522 | 6.242 | 6.362±1.105 |
| 30 | 5.184 | 3.828 | 5.036 | 4.683±0.744 |
| 60 | 2.990 | 2.780 | 3.542 | 3.104±0.394 |
| 120 | 1.631 | 1.783 | 2.431 | 1.948±0.425 |
| 240 | 0.612 | 0.808 | 0.860 | 0.760±0.131 |
| 480 | - | - | - | 0 |

As evident from the results shown in Tables 7 and 8, the transnasal formulation of the present invention containing fentanyl citrate exhibited a blood concentration comparable to the blood fentanyl concentration obtained by intravenous administration, indicating the extremely high absorption into the body. As demonstrated in FIG. 4 showing the kinetics of the plasma fentanyl concentration in relation to time in the transnasal administration and the intravenous administration, rise in the absorption curve into the body in the transnasal administration was comparable to that of the intravenous administration, and the kinetics of the blood fentanyl concentration was also equivalent to that of the intravenous administration. The results of the general observation also indicated the analgesic action of the formulation.

These results indicate that the transnasal formulation of the present invention is highly useful for cancer patients suffering from the serious pain.

### INDUSTRIAL APPLICABILITY

As described above, this invention provides an opioid analgesic composition for intranasal administration and a formulation for intranasal administration, which are provided with a markedly reduced risk of developing side effects compared to conventional oral, percutaneous, and transrectal formulations, and which exhibit prompt analgesic effects as well as excellent bioavailability. In particular, the prompt analgesic action after the administration attained by the intranasal administration is effective in swiftly ameliorating cancer pain of the terminal cancer patients and such action is effective not only for the continuous pain but also for the incident pain. In addition, reducing of constipation, nausea, emesis, and other side effects of the digestive system is extremely effective in controlling the pain, and omission of the therapeutic intervention such as injection enables use of the formulation by the patients themselves in a facility where no physician is present, namely, by a wider range of patients suffering from the pain. Accordingly, the medical benefits thus attained by the present invention are huge.

## Claims

1. A composition for nasal absorption comprising a carrier of calcium carbonate and/or calcium phosphate having an average particle size of 500 µm or less and an effective dose of an opioid analgesic uniformly distributed and attached to the carrier.

2. A composition for nasal absorption according to claim 1 wherein the opioid analgesic is a narcotic or a non-narcotic opioid analgesic.

3. A composition for nasal absorption according to claim 1 or 2 wherein the opioid analgesic is a narcotic opioid analgesic selected from opium, opium/ipecac preparation, morphine, morphine/atropine preparation, opium alkaloids preparation, opium alkaloids/atropine preparation, opium alkaloids/scopolamin preparation, ethylmorphine, oxycodon, oxycodon/atropine preparation, pethidine, pethidine/levallorphan preparation, codeine, dihydrocodeine, fentanyl, droperidol/fentanyl preparation, oxymetebanol, levorphanol, propoxyphene, methadone, hydromorphone, and meperidine; or a non-narcotic opioid analgesic selected from buprenorphine, butorphanol, pentazocine, pentazocine/naloxone preparation, dezocine, tramadol, and eptazocine; or a pharmaceutically acceptable salt thereof.

4. A composition for nasal absorption according to claim 1 or 2 wherein the opioid analgesic is a narcotic opioid analgesic selected from opium, opium/ipecac preparation, morphine hydrochloride, morphine sulfate, morphine hydrochloride/atropine sulfate preparation, opium alkaloids hydrochloride preparation, opium alkaloids hydrochloride/atropine sulfate preparation, opium alkaloids hydrochlorides/scopolamine hydrobromide preparation, ethylmorphine hydrochloride, compound oxycodone (oxycodone hydrochloride/hydrocotarnine hydrochloride), compound oxycodone/atropine sulfate preparation, pethidine hydrochloride, pethidine hydrochloride/levallorphan tartrate preparation, codeine phosphate, dihydrocodeine phosphate, fentanyl, fentanyl citrate, droperidol/fentanyl citrate preparation, oxymetebanol, levorphanol, propoxyphene, methadone, hydromorphone, and meperidine; or a non-narcotic opioid analgesic selected from buprenorphine hydrochloride, butorphanol tartrate, pentazocine, pentazocine hydrochloride, pentazocine hydrochloride/naloxone preparation, dezocine, tramadol hydrochloride, and eptazocine hydrobromide.

5. A composition for nasal absorption according to claim 1 or 2 wherein the opioid analgesic is morphine hydrochloride, morphine sulfate, morphine hydrochloride/atropine sulfate preparation, fentanyl, fentanyl citrate, droperidol/fentanyl citrate preparation, or buprenorphine hydrochloride, and the carrier is calcium carbonate.

6. A composition for nasal absorption according to any one of claims 1 to 5 wherein the carrier has an average particle size of 20 to 100 µm.

7. A composition for nasal absorption according to any one of claims 1 to 6 wherein content of the opioid analgesic is 0.01 to 50% by weight, and content of the carrier is 50 to 99.99% by weight.

8. A composition for nasal absorption according to any one of claims 1 to 7 wherein the opioid analgesic is an analgesic for post-surgery pain or cancer pain.

9. Use of a composition for nasal absorption in producing an analgesic for post-surgery pain or cancer pain, wherein said composition comprises a carrier of calcium carbonate and/or calcium phosphate having an average particle size of 500 µm or less and an effective dose of an opioid analgesic uniformly distributed and attached to the carrier.

10. A method for treating post-surgery pain or cancer pain comprising the step of intranasally administering a composition comprising a carrier of calcium carbonate and/or calcium phosphate having an average particle size of 500 µm or less and an effective dose of an opioid analgesic uniformly distributed and attached to the carrier.
